(19) Europäisches Patentamt
European Patent Office
Office européen des brevets

(11) Publication number: 0 137 276 B1

(12) EUROPEAN PATENT SPECIFICATION

(45) Date of publication of patent specification: 09.11.88

(51) Int. Cl.[4]: C 07 C 79/16, A 01 N 33/20 // C07C76/02, C07C47/228, C07C47/21

(21) Application number: 84110230.4

(22) Date of filing: 28.08.84

# (54) Soil fungicide.

(30) Priority: 29.08.83 JP 157675/83

(43) Date of publication of application: 17.04.85 Bulletin 85/16

(45) Publication of the grant of the patent: 09.11.88 Bulletin 88/45

(84) Designated Contracting States: BE DE FR GB IT LU NL

(56) References cited:
FR-A-1 328 303
US-A-2 332 482

CHEMICAL ABSTRACTS, vol.86, 1977, page 516, ref.no. 171039z; Columbus, Ohio, US; J. KOCHANY et al.: "Chemistry of nitroparaffins. Part CXVII. Phenylnitropropanols and their acetates."

(73) Proprietor: AGRO-KANESHO CO., LTD.
No. 4-1, Marunouchi 2-chome
Chiyoda-ku Tokyo (JP)

(72) Inventor: Watanabe, Syunnosuke
Room No. 3 Daimon-sou No. 1-1-19, Daimon-cho
Higashikurume-shi Tokyo (JP)
Inventor: Toyoda, Shigeaki
Room No. 6 Mochizuki-sou
No. 3-9-1, Umezono Kiyose-shi Tokyo (JP)

(74) Representative: Vossius & Partner
Siebertstrasse 4 P.O. Box 86 07 67
D-8000 München 86 (DE)

EP 0 137 276 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a fungicide for plants, particularly to an excellent soil fungicide having especially remarkable anti-fungous activity for the various types of pathogenic microorganisms that cause soil-borne diseases.

Soil-borne diseases and the prevention and disinfection thereof have received considerable attention recently among those engaged in agriculture, in order to solve cultivation problems as shown below:

(1) The successive cultivation of the same crop on the same ground results in specific soil diseases in the soil and makes it difficult to prevent the diseases and disinfect the soil.

(2) Because of an insufficient supply of compost and poor crop rotation, the physical, chemical and biological balance of the soil has been destroyed causing an increase in the infection potential of harmful microorganisms in the soil.

(3) Microorganisms themselves have developed resistance against the preventive agents, which makes the prevention and disinfection more difficult.

(4) From the pharmaceutical viewpoint, the toxicity of soil-antiseptics and fungicides, such as chloropicrin, pentachloronitrobenzene, etc., that have conventionally been used, and the impurities mixed therein, such as hexachloro cyclopentadiene (HCP) mixed in pentachloro nitrobenzene, have caused serious problems; accordingly, there is a demand for chemicals which are safer and easier to handle. (Chloropicrin is strongly lachrymatory and is inconvenient to handle.)

(5) There is a demand for chemicals which require a shorter time for soil disinfection and which allow planting and sowing within a short time after disinfection, without subjecting the crops to any phytotoxicity. Conventional soil antiseptics require more than a week for the disinfection, and after the disinfection of the soil, the ground has to be left for another seven to ten days in order to prevent the crops from phytotoxicity, by thoroughly releasing any gas remaining in the soil and, further still by thoroughly turning over the soil for aeration.

Against this background as mentioned above, there is a call throughout the world for ideal soil fungicides.

Thus, the technical problem underlying the present invention is to develop an ideal soil fungicide free of such defects as those of the conventional soil fungicides. This problem is solved by providing novel nitro alcohol derivatives represented by the general formula:

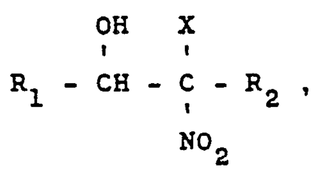

wherein $R_1$ represents

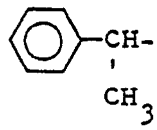 , 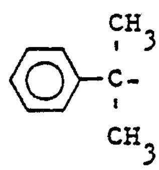

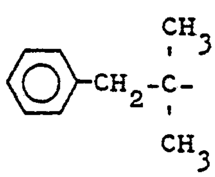 , 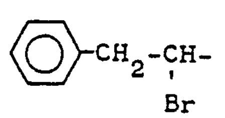 , 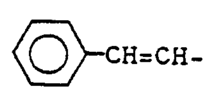 ,

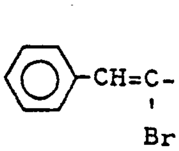 , or 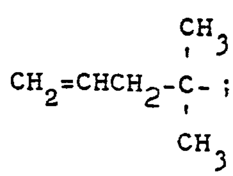 ;

X represents a hydrogen atom, an alkyl group having 1—4 carbon atoms or a halogen atom; $R_2$ represents a hydrogen atom or an alkyl group having 1—4 carbon atoms.

The usefulness of the soil fungicide of the present invention may be summarized as follows:

(1) It is effective for various types of diseases in soil.

For damping-off, for example, there is no soil fungicide effective for both Pythium spp. and Rhizoctonia spp. Mepronil (3′-isopropoxy-2-methyl-benzanilide), for example, is effective on Rhizoctonia spp. but has no effect on Pythium spp. On the other hand, hydroxyisoxazol is effective on Pythium spp. but has little effect on Rhizoctonia spp. Pentachloronitrobenzene is effective on Rhizoctonia spp. but has little effect on

Pythium spp. Thus, a satisfactory disinfection of damping-off cannot be exerted unless these chemicals are mixed together. Furthermore, there are still fewer chemicals effective for other serious soil diseases, such as Plasmodiophora brassicae, in the present circumstances, pentachloronitrobenzene and tetrachloroisophthalonitrile are somewhat effective, however, not effective enough. Compared with these fungicides, it has been confirmed that the soil fungicide of the present invention is not only effective on the damping-off fungi, both Pythium spp. and Rhizoctonia spp., but also exert an excellent effect on Plasmodiophora brassicae, as shown in Examples 1 and 2 of the present application.

(2) The soil fungicide of the present invention makes it possible to carry out the disinfection within a short time and proceed with planting without delay.

As is the case with such soil fungicide, as chloropicrin or Dazomet (3,5-Dimethyl tetrahydro-1,3,5(2H)-thiadiazine-2-thione), a rather long time period is required between the disinfection of the soil and the following planting for the safety of the crops. In the case of chloropicrin or Dazomet seven to ten days are required for disinfection after the treatment of the soil, and planting cannot be completed without incurring the risk of intoxicating the crops unless the soil has been left for seven to ten days prior to the planting. Particularly, when the treatment is carried out in early spring, or it is performed in such low-temperature areas as Europe, this time period must be extended further, sometimes to several months. This is a serious problem which causes a great deal of inconveniences in farming operations as well as in the utilization of land. If, however, the soil fungicide of the present invention is used, all the operations of disinfection treatment, planting and sowing can be completed within a short time, e.g. two to three days, as shown in Examples 1, 2, 3 and 4, which is a great contribution to farming operations. Furthermore, in the case of many soil fungicides, aerating operation to release the remaining active gaseous substances is required after completion of the disinfection treatment, and planting free of phytotoxicity is only possible after completion of the aeration. In the case of the soil fungicide of the present invention, on the other hand, no such aerating operation is required, as shown in Examples 2 and 4 and thus it provides a good deal of convenience and simplification in farming operations.

(3) The soil fungicide of the present invention is highly safe for man and beast and has no lachrymatory and skin-irritant effects.

The soil fungicides that are most widely used today, such as chloropicrin and methylbromide, are violent poisons; these substances have violently irritative fumes as well as skin-irritant and lachrymatory properties. Particularly, in suburban cultivation areas they are causing serious pollution problems. The soil fungicide of the present invention, since it is of low toxicity and has scarcely any such irritant fumes or skin irritant properties, is quite advantageous in farming work.

The nitroalcohol derivatives to be used as the active ingredient of the soil fungicide of the present invention are compounds that can be prepared according to the following process, and are all novel compounds. That is to say, they can be formed by aldol-condensing nitromethane and aldehyde in the presence of alkali; or by brominating the compound obtained by aldol condensation. In addition, said aldehyde to be used for the preparation of these compounds can be synthesized by general synthesizing methods, or the method of Hans K. Dietl et al., i.e. the method disclosed in Tetrahedron Letters, (15), 1273—1275, (1973).

The reactions involved in the production of the compounds of the present invention proceed according to the following reaction formula:

1. Production of nitro alcohol compounds.

$$R_1CHO = CH_3NO_2 \xrightarrow[H^+]{NaOH/CH_3OH} R_1\text{—}\underset{|}{\overset{OH}{CH}}\text{—}CH_2NO_2$$

2. Production of brominated nitro alcohol compounds.

(1) Bromination of

$$R_1\text{—}\overset{OH}{CH}\text{—}CH_2NO_2$$

$$R_1\overset{OH}{CH}\text{—}CH_2NO_2 \xrightarrow[Br_2]{NaOH/CH_3OH} R_1\text{—}\overset{OH}{CH}\text{—}CHBrNO_2$$

(2) Reaction of $R_1CHO$ and $CH_3NO_2$

$$R_1CHO + CH_3NO_2 \xrightarrow[Br_2]{NaOH/CH_3OH} R_1\text{—}\overset{OH}{CH}\text{—}CHBrNO_2$$

Hereinafter, certain examples for producing the compounds of the present invention will be described.

Preparation Example 1

2,2-dimethyl-3-phenylpropion aldehyde

Into a three-necked flask were put 140 ml of water, 140 g of sodium hydroxide, 200 ml of benzene, and 14.7 g of tetrabutyl ammonium iodide, and these were then heated and agitated at 70°C. Subsequently, a mixture consisting of 380 g of benzyl chloride, and 288 g of isobutyl aldehyde was dripped thereinto over a 5-hour period. Heating and agitation at 70°C was proceeded for further 2 hours, then the benzene layer was separated. After rinsing with water, it was dried with anhydrous magnesium sulfate. After the solvent was distilled off, it was subjected to distillation under reduced pressure. The obtained amount was 364 g (yield 75%), which had a boiling point of 95°C (7.2 mm Hg).

Preparation Example 2

1-nitro-3,3-dimethyl-4-phenyl-2-butanol

(Compound No. 6)

One hundred grams of 2,2-dimethyl-3-phenyl propion aldehyde, obtained in Preparation Example 1, and 37 g of nitromethane were dissolved in 500 ml of methanol. The solution was then cooled, and 61 ml of 10N aqueous sodium hydroxide solution was introduced thereinto under agitation; the reaction mixture was then agitated for 30 minutes, and neutralized with an excess amount of 20% hydroxyl amine hydrochloride aqueous solution. The solution was extracted with ether, and after washing with aqueous sodium hydrogensulfite solution and water, it was dried with anhydrous magnesium sulfate. The solvent was distilled off, thereby obtaining the objective product. The amount obtained was 137.5 g (yield 98%).

Preparation Example 3

1-bromo-nitro-3,3-dimethyl-4-phenyl-2-butanol

(Compound No. 7)

22.3 g of the 1-nitro-3,3-dimethyl-4-phenyl-2-butanol, obtained in Preparation Example 2, were added to 100 ml of methanol containing 2.3 g of sodium, while being cooled and agitated. After agitation for 30 minutes, 20 ml of carbon tetrachloride containing 16 g of bromine were introduced thereinto so as to maintain the temperature at 0°C. After 1 hour agitation, methanol was distilled off under reduced pressure. One hundred milliliters of ether were added to the reaction mixture, and after washing it three times with water, the mixture was dried with anhydrous magnesium sulfate. The solvent was distilled off to obtain the object product. The amount obtained was 29.8 g (yield 98.6%).

Preparation Example 4

2,2-dimethyl-4-pentene-1-al

Synthesis was carried out in the same manner as Preparation Example 1, except that 76.5 g of allyl chloride and 96 g of isobutyl aldehyde were used as the starting material and a reaction temperature of 60°C was employed. The amount of the product obtained was 67.2 g (yield 60%).

Preparation Example 5

1-bromo-1-nitro-3,3-dimethyl-5-hexane-2-ol

(Compound No. 13)

1-nitro-3,3-dimethyl-5-hexene-2-ol was synthesized in the same manner as Preparation Example 2 by using 11.2 g of aldehyde formed in Preparation Example 4, 6.1 g of nitromethane and 10 ml of 10N sodium hydroxide aqueous solution as the starting material (amount obtained was 16.3 g (yield 94.7%)). Ten grams of the synthesized material was dissolved in methanol containing 1.34 g of sodium, and the object product was synthesized in the same manner as Preparation Example 3, by using 9.4 g of bromine. The amount obtained was 1.38 g (yield 94.5%).

The following table 1 shows the physical properties of the active ingredient compound of the present invention.

Table 1

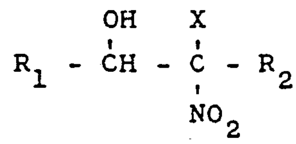

| Compound No. | $R_1$ | $R_2$ | X | Infrared spectrum($cm^{-1}$) | | |
|---|---|---|---|---|---|---|
| 1 | 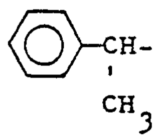 | H | H | 1600 | 1555 | 3460 |
| 2 | " | H | Br | 1600 | 1565 | 3450 |
| 3 | " | H | $CH_3$ | 1600 | 1550 | 3450 |
| 4 | 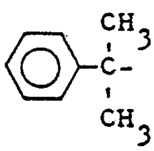 | H | H | 1600 | 1560 | 3420 |
| 5 | " | H | Br | 1600 | 1565 | 3415 |
| 6 | 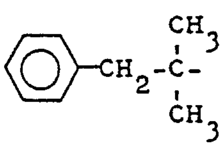 | H | H | 3410 | 1600 | 1590 |
| 7 | " | H | Br | 3405 | 1600 | 1585 |
| 8 | " | $CH_3$ | Br | 3910 | 1600 | 1575 |
| 9 | 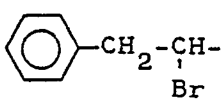 | H | H | 1600 | 1560 | 3420 |
| 10 | " | H | Br | 1600 | 1565 | 3415 |
| 11 | 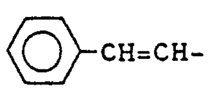 | H | Br | 3390 | 1620 | 1555 |
| 12 | 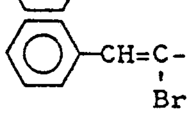 | H | Br | 1600 | 3430 | 1560 |
| 13 | 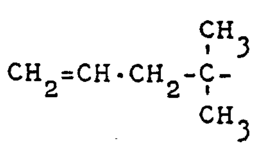 | H | Br | 1640 | 3420 | 1555 |

The soil fungicides of the present invention may be used by mixing the active ingredient as it is in the surface soil of the ground, but it may also be used by dissolving or dispersing it in various solvents, such as acetone, xylene, etc., or in vegetable oils, paraffins, etc. Further, it is also possible to use them by mixing with such inorganic or organic carriers as diatom earth, urea, sawdust, etc. Still further, they may be used by adding thereto various types of adjuvants, such as various surfactants, or lubricants, such as white carbon, etc. Hereinafter certain concrete examples of these preparations will be described.

Example 1

Emulsifiable concentrate

Twenty parts of the compound No. 7 were dissolved in 75 parts of xylene, and as the emulsifier, 2 parts of polyoxyethylene alkyl allylether, 1 part of polyoxyethylene alkylether, and 2 parts of alkylbenzene sulfonate were mixed therewith and dissolved, thereby obtaining an emulsifiable concentrate. Said emulsifiable concentrate is applied to the soil, after being diluted with water.

Example 2

Granules

Three parts of the compound No. 2 and 5 parts diatom earth were thoroughly mixed and pulverized. To this mixture, 56 parts of clay, 30 parts of bentonite, 3 parts of sodium ligninsulfonate and 1 part of alkylbenzene sodium sulfonate were added and the materials were thoroughly mixed, then after adding water thereto, the mixture was granulated by an extrusion-granulator, dried, and then subjected to particle-regulation; a granular preparation was thereby obtained. The preparation is applied directly to the soil.

Example 3

Oil preparation

Fifteen parts of the compound No. 13 were dissolved in 85 parts of kerosine to form an oil preparation. The preparation is directly applied to the soil.

The active ingredient compounds of the composition of the present invention may be used, if necessary, by blending with such fungicide as pentachloronitrobenzene (PCNB), bis(dimethylthiocarbamoyl)disulfide, tetrachloroisophthalonitrile, 3-hydroxy-5-methylisoxazol, 5-ethoxy-3-trichloromethyl-1,2,4-thiadiazol, N-(trichloromethylthio)-4-cyclohexene-1,2-dicarboxyimide, N-(1,1,2,2-tetrachloroethylthio)-4-cyclohexene-1,2-dicarboxyimide, chloropicrin, etc., such soil fungicides as 1,2-dibromoethane, 1,3-dichloropropane, 1,2-dichloropropane, bis(2-chloro-1-methylethyl)-ether, bromomethane, ammonium-(sodium)N-methyldithiocarbamate, etc., nematocides, organo phosphorus type insecticides, carbamate type insecticides, chlorine type insecticides, or various types of herbicides, fertilizers, etc. or they may be used in mixture with these substances.

Hereinafter the effects of the soil fungicides of the present invention will be described in Test Examples.

Test Example 1

Diseases in the Sample Soil: Clubroot of cruciferous green vegetables (Plasmodiophora brassicae)

Sample Crop: Chinese cabbage

Testing method:

About 1.5 l of disinfected soil, which was inoculated so as to provide a soil having a dormant spore density of $1 \times 10^6$ ml, was placed in plastic pots of diameter 14 cm, and these samples were treated with the designated amount of the chemicals. That is to say, each of the compounds to be used in the composition of the present invention were prepared as a 20% emulsifiable concentrate, by means of which soil drenching treatment was given so as to provide 8 kg or 4 kg of the active ingredient per 10 ares. Said soil drenching treatment was effected by dissolving the designated amount of emulsifiable concentrate in 2000 l of water per 10 ares and thoroughly applying it to the surface of the soil. Three days after the treatment the soil was aerated and on the next day Chinese cabbage seeds were sown, 5 seeds per pot. Forty days after the chemical treatment the fresh weight of the above-ground parts of the plants was measured and the degree of gall-formation was evaluated. The evaluation of the gall-formation degree was made according to the method described by Williams, et al., in "Phytopathology" *56,* 624-626 (1966). That is to say, observing with the naked eye, the gall-formation degree was classified into the following:

O : Normal
I : Slight formation of gall on lateral roots.
II : Medium formation of gall on lateral or tap roots.
III : Severe formation of gall on lateral or tap roots.

The gall-formation degree was obtained by use of the above classification and according to the following formula:

Gall-formation degree =

$$\frac{\begin{pmatrix}\text{No. of plants}\\ \text{of rating}\\ \text{I} \times 10\end{pmatrix} + \begin{pmatrix}\text{No. of plants}\\ \text{of rating}\\ \text{II} \times 80\end{pmatrix} + \begin{pmatrix}\text{No. of plants}\\ \text{of rating}\\ \text{III} \times 100\end{pmatrix}}{\text{Total No. of Plants observed}}$$

The fresh weight of the above-ground parts of the plants is the average value of ten plants. Layout of the Plot: Each set was of 2 pots, in total 10 plants.

Test Result:

The following Table 2 shows the gall-formation degree and the percentage of the fresh weight of the above-ground parts of the plants compared with non-inoculated plants. The percentage of the fresh weight of the above-ground parts of the plants compared with non-inoculated plants is the numerical value indicated by percentage of the fresh weight of the above-ground parts in the treated sections compared with that of the non-inoculated and non-treated sections.

Table 2

| Composition of the present invention | | Dosage | Gall-forming | Percent of | Phytotoxicity |
|---|---|---|---|---|---|
| Compound No. | Preparation | a.i.kg/10 ares | degree | fresh weight | |
| 1 | 20% EC | 8 | 2 | 98 | - |
| | | 4 | 2 | 102 | - |
| 2 | " | 8 | 0 | 125 | - |
| | | 4 | 4 | 117 | - |
| 3 | " | 8 | 0 | 100 | - |
| | | 4 | 3 | 96 | - |
| 4 | " | 8 | 2 | 102 | - |
| | | 4 | 8 | 103 | - |
| 5 | " | 8 | 0 | 121 | - |
| | | 4 | 0 | 133 | - |
| 6 | " | 8 | 1 | 98 | - |
| | | 4 | 7 | 101 | - |
| 7 | " | 8 | 1 | 107 | - |
| | | 4 | 4 | 97 | - |
| 8 | " | 8 | 2 | 115 | - |
| | | 4 | 7 | 107 | |
| 9 | " | 8 | 0 | 106 | - |
| | | 4 | 1 | 104 | - |
| 10 | " | 8 | 0 | 112 | - |
| | | 4 | 0 | 104 | - |

Table 2 (cont'd)

| Composition of the present invention: Compound No. | preparation | Dosage a.i.kg/10 ares | Gall-forming degree | Percent of fresh weight | Phytotoxicity |
|---|---|---|---|---|---|
| 11 | 20% EC | 8 | 0 | 114 | - |
| | | 4 | 0 | 93 | - |
| 12 | " | 8 | 0 | 89 | - |
| | | 4 | 0 | 105 | - |
| 13 | " | 8 | 5 | 111 | - |
| | | 4 | 12 | 90 | - |
| Pentachloronitrobenzene (PCNB) (Reference) | | 8 | 7 | 80 | - |
| | | 4 | 45 | 69 | - |
| Non-treatment with fungal inoculation | | - | 100 | 25 | - |
| Non-treatment with no fungal inoculation | | - | 0 | 100 | - |

EC: Emulsifiable Concentrate

Test Example 2
Disease in the Sample Soil: Damping-off fungus of cucumber seedlings (Pythium aphanidermatum) (Rhizoctonia solani)
Sample Crop: Cucumber (variety: Sagami Hanjiro)
Testing Method:

About 300 ml of disinfected soil, which was inoculated with the damping-off fungi, was packed in porcelain pots of diameter 9 cm and treated with the respective chemicals. That is to say, the respective compounds to be used for the composition of the present invention were each prepared as a 20% emulsifiable concentrate, by means of which soil drenching treatment was carried out so as to provide the proportion of the active ingredient of 5 kg or 1 kg per 10 ares. The soil drenching treatment was performed by dissolving the designated amount of the emulsifiable concentrate in 3000 l of water per 100 ares and applying it thoroughly to the surface of the soil. Three days after the treatment, cucumber seeds were sown, 10 seeds to a pot. Aeration was not made. Two weeks after sowing, the number of germination seeds, the number of damping-off plants and the number of brown-colored plants on the basal parts were recorded, and the infection percentage was obtained as follows:

$$\text{Infection\%} = \frac{\begin{pmatrix}\text{No. of non-}\\ \text{germination}\\ \text{seeds}\end{pmatrix} + \begin{pmatrix}\text{No. of}\\ \text{damping-}\\ \text{off plants}\end{pmatrix} + \begin{pmatrix}\text{No. of brown-}\\ \text{coloured plants on}\\ \text{the basal parts}\end{pmatrix}}{\text{Total seeds in sample}} \times 100$$

Layout of the plot: Each set was of 2 pots, in total 10 plants.
Test Result: Table 3 shows the rate of infection (%).

Table 3

| Composition of the present invention | | Dosage | Infection rate % | | Phytotoxicity |
|---|---|---|---|---|---|
| Compound No. | Preparation | a.i.kg/10 ares | P.aphanidermatum | R.solani | |
| 1 | 20% EC | 5 | 0 | 0 | - |
| | | 1 | 0 | 0 | - |
| 2 | " | 5 | 0 | 0 | - |
| | | 1 | 0 | 0 | - |
| 3 | " | 5 | 0 | 0 | - |
| | | 1 | 0 | 0 | - |
| 4 | " | 5 | 0 | 0 | - |
| | | 1 | 0 | 0 | - |
| 5 | " | 5 | 0 | 0 | - |
| | | 1 | 0 | 0 | - |
| 6 | " | 5 | 0 | 0 | - |
| | | 1 | 0 | 0 | - |
| 7 | " | 5 | 0 | 0 | - |
| | | 1 | 0 | 0 | - |
| 8 | " | 5 | 0 | 0 | - |
| | | 1 | 0 | 0 | - |
| 9 | " | 5 | 0 | 0 | - |
| | | 1 | 0 | 0 | - |
| 10 | " | 5 | 0 | 0 | - |
| | | 1 | 0 | 0 | - |

Table 3 (con'd)

| Composition of the present invention | | Dosage | Infection rate % | | Phytotoxicity |
|---|---|---|---|---|---|
| Compound No. | Preparation | a.i.kg/10 ares | P.aphanidermatum | R.solani | |
| 11 | 20% EC | 5 | 0 | 0 | - |
| | | 1 | 0 | 0 | - |
| 12 | " | 5 | 0 | 0 | - |
| | | 1 | 0 | 0 | - |
| 13 | " | 5 | 0 | 0 | - |
| | | 1 | 0 | 0 | - |
| Captan (Reference) | 80% WP | 2 | 25 | 5 | - |
| Non-treatment with fungal inoculation | | - | 100 | 85 | - |
| Non-treatment with no fungal inoculation | | - | 0 | 0 | - |

-------------------------------------

WP: Wettable powder

EC: Emulsifiable Concentrates

Captan: N-(Trichloromethylthio)-4-cyclohexene-1,2-dicarboximide

Test Example 3

The relation between the time period from the treatment of soil until sowing and phytotoxicity was examined. Soil was packed in porcelain pots of diameter 9 cm, and soil drenching treatment was performed so as to make the proportions of the respective chemicals 10 kg or 5 kg per 10 ares. The amount of water drenched was in the proportion of 3000 l per 10 ares. One, three, seven and fourteen days after the treatment, 10 seeds of Chinese cabbage were sown in each pot, 20 seeds in total for a set of two pots, and germinating rate was examined. The result is shown in Table 4. Said germinating rate was expressed as follows:

$$\text{Germinating rate} = \frac{\text{No. of germinated seeds}}{\text{Total No. of seeds}} \times 100$$

Table 4

| Compound No. | Preparation | Dosage a.i.kg/10 ares | Days between treatment and sowing 1 | 3 | 7 | 14 |
|---|---|---|---|---|---|---|
| 1 | EC | 10 | 10* | 100 | 100 | 100 |
| | | 5 | 60 | 100 | 100 | 100 |
| 2 | EC | 10 | 5 | 100 | 100 | 100 |
| | | 5 | 35 | 100 | 100 | 100 |
| 3 | EC | 10 | 20 | 100 | 100 | 100 |
| | | 5 | 45 | 100 | 100 | 100 |
| 4 | EC | 10 | 15 | 100 | 100 | 100 |
| | | 5 | 20 | 100 | 100 | 100 |
| 6 | EC | 10 | 20 | 100 | 100 | 100 |
| | | 5 | 65 | 100 | 100 | 100 |
| Dazomet (Reference) | 10% EC | 10 | 0 | 0 | 0 | 100 |
| | | 5 | 0 | 0 | | 100 |
| Chloro-picrin | oil | 10 | 0 | 0 | 35 | 100 |
| | | 5 | 0 | 0 | 60 | 100 |

* Figures show germination percentage.

EC : Emulsifiable Concentrate

Test Example 4

After the disinfection treatment of the soil, the influence on the effectiveness with or without soil sealing was examined. Soil inoculated with damping-off fungi (Pythium aphanidermatum) of cucumber was packed in 1/2000 Wagner are pots, which were then subjected to soil drenching treatment with a designated amount of the water-diluted solution of the active ingredient compound of the composition of the present invention, then the soil was thoroughly incorporated. For comparison, chloropicrin was injected at a depth of 15 cm at the center of a pot. For each treatment, the pots were divided into a sealing plot and a non-sealing plot, and in the sealing plot, the upper surface of each pot was mulched completely with a thick vinyl sheet to prevent dispersal of gases. Ten days after the treatment, the sealing was removed; and two days after, the cucumber seeds (variety: Sagami Hanjiro) were sown 20 seeds to a pot; fourteen days after the sowing, the number of germinated seeds and that of damping-off were recorded. The following Table 5 shows the germinating rate, rate of damping-off, and that of healthy plants. Said germinating rate, rate of damping-off, and that of healthy plants were numerical values obtained by the following formulae, respectively:

$$\text{Germinating rate} = \frac{\text{No. of germinated seeds}}{\text{Total no. of seeds}} \times 100$$

$$\text{Rate of damping off} = \frac{\text{No. of infected plants}}{\text{Total no. of seeds}} \times 100$$

$$\text{Rate of healthy plants} = \frac{\begin{array}{c}\text{No. of healthy plants}\\ \text{(infected plants excluded)}\end{array}}{\text{Total no. of seeds}} \times 100$$

**Table 5**

| Compound No. | Prepa-ration | With or without mulching | Dosage a.i.kg/10 ares | Germination rate | Rate of damping-off infection | Rate of healthy plants |
|---|---|---|---|---|---|---|
| 1 | WP | With | 5 | 100 | 0 | 100 |
| | | | 1 | 95 | 0 | 95 |
| | | Without | 5 | 100 | 0 | 100 |
| | | | 1 | 100 | 0 | 100 |
| 4 | WP | With | 5 | 100 | 0 | 100 |
| | | | 1 | 100 | 0 | 100 |
| | | Without | 5 | 100 | 0 | 100 |
| | | | 1 | 100 | 0 | 100 |
| 6 | WP | With | 5 | 95 | 0 | 95 |
| | | | 1 | 100 | 0 | 100 |
| | | Without | 5 | 100 | 0 | 100 |
| | | | 1 | 100 | 0 | 100 |
| Chloropicrin (Reference) | Oil | With | 5 | 100 | 0 | 100 |
| | | | 1 | 100 | 0 | 100 |
| | | Without | 5 | 75 | 10 | 65 |
| | | | 1 | 10 | 10 | 0 |
| Untreated | | With | - | 0 | 0 | 0 |
| | | Without | - | 5 | 5 | 0 |

WP : Wettable Powder

## Claims

1. Nitroalcohol derivatives represented by the general formula:

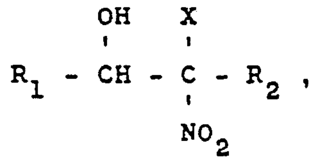

wherein $R_1$ represents

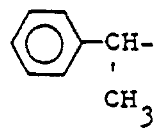 , 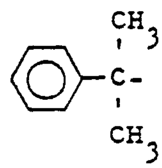

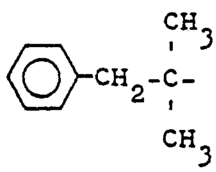 , 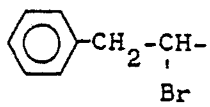 , 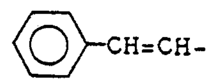 ,

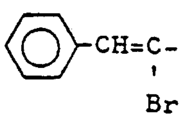 , or 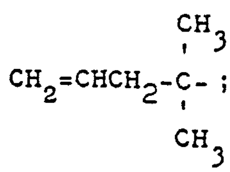 ;

X represents a hydrogen atom, an alkyl group having 1—4 carbon atoms or a halogen atom; $R_2$ represents a hydrogen atom or an alkyl group having 1—4 carbon atoms.

2. A compound according to Claim 1, which is 1-nitro-3,3-dimethyl-4-phenyl-2-butanol.

3. A compound according to Claim 1, which is 1-bromo-1-nitro-3,3-dimethyl-4-phenyl-2-butanol.

4. The compound according to Claim 1, which is 1-bromo-1-nitro-3,3-dimethyl-5-hexene-2-ol.

5. A soil fungicide characterized in that it contains as its active ingredient a nitroalcohol derivative represented by the general formula:

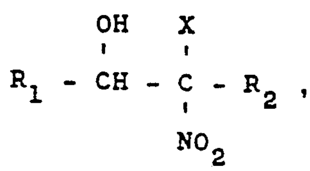

wherein $R_1$ represents

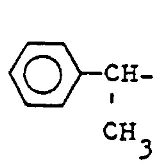 , 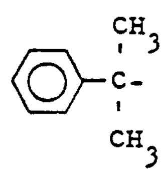

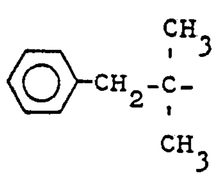 , 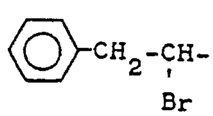 , 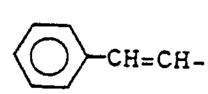 ,

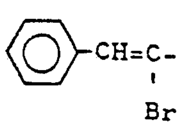 , or 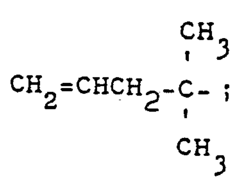 ;

X represents a hydrogen atom, an alkyl group having 1—4 carbon atoms or a halogen atom; $R_2$ represents a hydrogen atom or an alkyl group having 1—4 carbon atoms.

6. The soil fungicide according to Claim 5, which contains 1-nitro-3,3-dimethyl-4-phenyl-2-butanol as its active ingredient.

7. The soil fungicide according to Claim 5, which contains 1-bromo-1-nitro-3,3-dimethyl-4-phenyl-2-butanol as its active ingredient.

8. The soil fungicide according to Claim 5, which contains 1-bromo-1-nitro-3,3-dimethyl-5-hexane-2-ol as its active ingredient.

**Patentansprüche**

1. Nitroalkoholderivate der allgemeinen Formel:

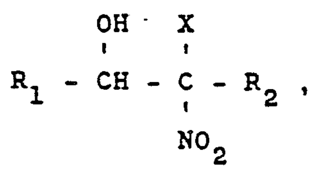

in der $R_1$

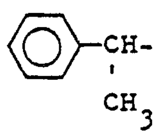 , 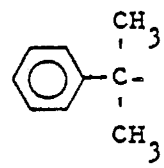

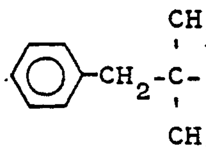 , 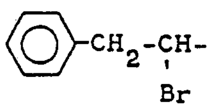 , 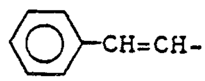 ,

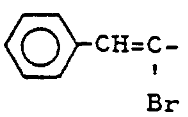 , oder 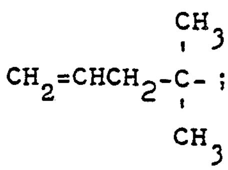

darstellt;
X ein Wasserstoffatom, einen Alkylrest mit 1 bis 4 Kohlenstoffatomen oder ein Halogenatom bedeutet; $R_2$ ein Wasserstoffatom oder einen Alkylrest mit 1 bis 4 Kohlenstoffatomen bedeutet.

2. Verbindung nach Anspruch 1, welche 1-Nitro-3,3-dimethyl-4-phenyl-2-butanol ist.

3. Verbindung nach Anspruch 1, welche 1-Brom-1-nitro-3,3-dimethyl-4-phenyl-2-butanol ist.

4. Verbindung nach Anspruch 1, welche 1-Brom-1-nitro-3,3-dimethyl-5-hexen-2-ol ist.

5. Bodenpilztötendes Mittel, dadurch gekennzeichnet, daß es als aktiven Bestandteil ein Nitroalkoholderivat der allgemeinen Formel

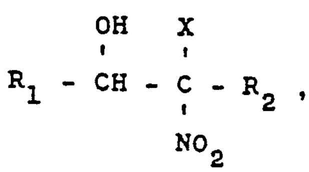

in der $R_1$

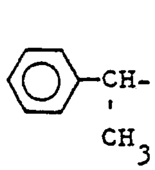 , 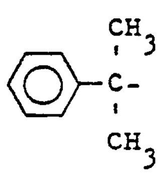 ,

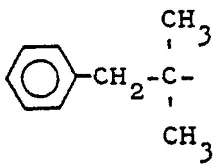,
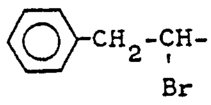,
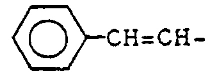,

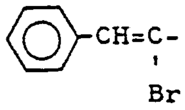, oder
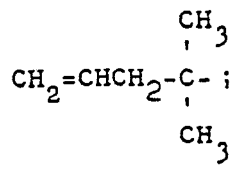;

darstellt;
X ein Wasserstoffatom, einen Alkylrest mit 1 bis 4 Kohlenstoffatomen oder ein Halogenatom bedeutet; $R_2$ ein Wasserstoffatom oder einen Alkylrest mit 1 bis 4 Kohlenstoffatomen bedeutet.

6. Bodenpilztötendes Mittel nach Anspruch 5, das 1-Nitro-3,3-dimethyl-4-phenyl-2-butanol als aktiven Bestandteil enthält.

7. Bodenpilztötendes Mittel nach Anspruch 5, das 1-Brom-1-nitro-3,3-dimethyl-4-phenyl-2-butanol als aktiven Bestandteil enthält.

8. Bodenpilztötendes Mittel nach Anspruch 5, das 1-Brom-1-nitro-3,3-dimethyl-5-hexen-2-ol als aktiven Bestandteil enthält.

**Revendications**

1. Dérivés nitroalcooliques représentés par la formule générale:

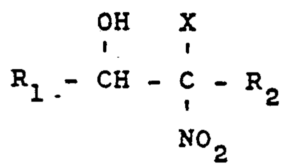,

dans laquelle $R_1$ représente

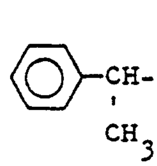,
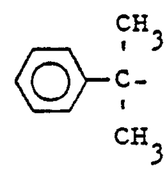

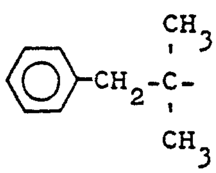,
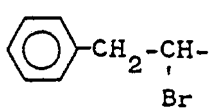,
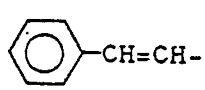,

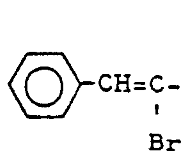, ou
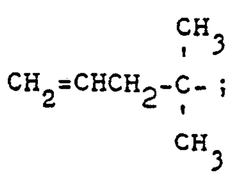;

X représente un atome d'hydrogène, un groupe alkyle comportant 1 à 4 atomes de carbone ou un atome d'halogène; $R_2$ représente un atome d'hydrogène ou un groupe alkyle comportant 1 à 4 atomes de carbone.

2. Composé suivant la revendication 1, caractérisé en ce qu'il est constitué par le 1-nitro-3,3-diméthyl-4-phényl-2-butanol.

3. Composé suivant la revendication 1, caractérisé en ce qu'il est constitué par le 1-bromo-1-nitro-3,3-diméthyl-4-phényl-2-butanol.

4. Composé suivant la revendication 1, caractérisé en ce qu'il est constitué par le 1-bromo-1-nitro-3,3-diméthyl-5-hexène-2-ol.

5. Fongicide du sol, caractérisé en ce qu'il contient comme ingrédient actif un dérivé nitro alcoolique représente par la formule générale:

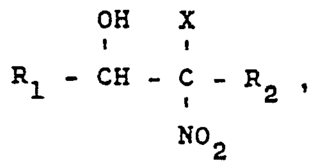

dans laquelle $R_1$ représente un groupe

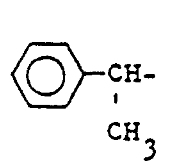 , 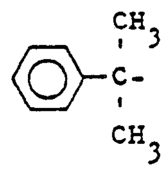

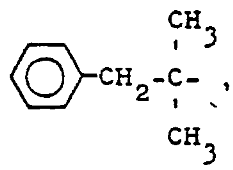 , 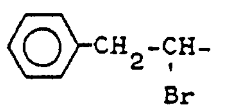 , 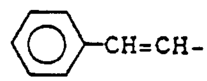 ,

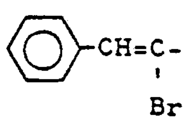 , ou 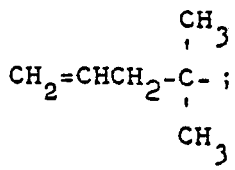 ;

X représente un atome d'hydrogène, un groupe alkyle comportant 1 à 4 atomes de carbone ou un atome d'halogène et $R_2$ représente un atome d'hydrogène ou un groupe alkyle comportant 1 à 4 atomes de carbone.

6. Fongicide de sol suivant la revendication 5, caractérisé en ce qu'il contient du 1-nitro-3,3-diméthyl-4-phényl-2-butanol comme ingrédient actif.

7. Fongicide du sol suivant la revendication 5, caractérisé en ce qu'il contient du 1-bromo-1-nitro-3,3-diméthyl-4-phényl-2-butanol comme ingrédient actif.

8. Fongicide du sol suivant la revendication 5, caractérisé en ce qu'il contient du 1-bromo-1-nitro-3,3-diméthyl-5-hexène-2-ol comme ingrédient actif.